# EUROPEAN PATENT SPECIFICATION

(11) **EP 2 698 146 B1**
(45) Date of publication and mention of the grant of the patent: **14.08.2019**
(21) Application number: 13180688.7
(22) Date of filing: 16.08.2013
(51) Int. Cl.: A61K 9/00, A61K 31/138, A61K 31/4985, A61K 9/20

(54) **Tablet Formulations Comprising Tadalafil and Dapoxetine**
Tablettenformulierungen mit Tadalafil und Dapoxetin
Formulations de comprimé comprenant du tadalafil et diapoxetine

(30) Priority: 17.08.2012 TR 201209599; 31.10.2012 TR 201212488
(43) Date of publication of application: 19.02.2014
(73) Proprietor: Sanovel Ilac Sanayi ve Ticaret A.S., 34460 Istanbul (TR)
(72) Inventor: Türkyilmaz, Ali, 34460 Istanbul (TR); Okyar, Isin Rukiye, 34460 Istanbul (TR)
(74) Representative: Sevinç, Erkan

(56) References cited:
- EP-A1- 2 316 435
- WO-A2-2008/146178
- "Tadapox", RSMenterprises , 1 December 2010 (2010-12-01), XP002702895, Retrieved from the Internet: URL:http://trade.indiamart.com/details.mp? offer=2091554055 [retrieved on 2013-07-15]
- "T-Ject 60", Potency Pills , 23 February 2012 (2012-02-23), XP002702896, Retrieved from the Internet: URL:http://pills-impotence.com/t-ject-60-i s-the-newest-brand-medication-tadalafil-an d-dapoxetine/ [retrieved on 2013-07-15]
- DRESSER M J ET AL: "Dapoxetine, a novel treatment for premature ejaculation, does not have pharmacokinetic interactions with phosphodiesterase-5 inhibitors", INTERNATIONAL JOURNAL OF IMPOTENCE RESEARCH, STOCKTON, BASINGSTOKE, GB, vol. 18, no. 1, 1 January 2006 (2006-01-01), pages 104-110, XP009130077, ISSN: 0955-9930

## Description

### Technical Field

The present invention relates to a tablet formulation comprising a combination of dapoxetine or a pharmaceutically acceptable salt thereof with tadalafil or a pharmaceutically acceptable salt thereof. The present invention also relates to a process for preparing such a formulation and to the use thereof in the treatment of premature ejaculation.

### Background of Invention

Selective serotonin reuptake inhibitors (SSRI) are used in the long-term prophylaxis of many types of depression, including the endogenous type, recurrent depression, and in the treatment of obsessive-compulsive disorders, panic attack, social phobias, and the bulimia nervosa disease. Dapoxetine, which was first disclosed in the European patent publication EP 0288188 B1 is a selective serotonin reuptake inhibitor. Dapoxetine is used for the treatment of depression and premature ejaculation and has a chemical structure shown with Formula I. Additionally, dapoxetine was approved in Switzerland and in Finland for use in the treatment of premature ejaculation.

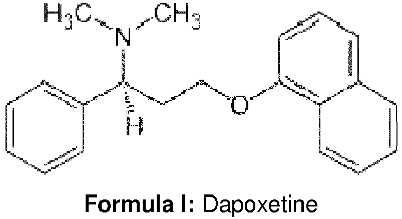

Following oral administration, dapoxetine is rapidly absorbed and rapidly enters the circulation by almost completely binding to plasma proteins. Therefore, it achieves the peak plasma concentration (Cₘₐₓ) in 1 hour following oral administration. Orally-administered tablets of dapoxetine are commercially available under the name Priligy®, comprising 30 mg or 60 mg dapoxetine hydrochloride per tablet, as well as excipients including lactose monohydrate, microcrystalline cellulose, croscarmellose sodium, colloidal anhydrous silica, magnesium stearate, hypromellose, titanium dioxide (E171), triacetin, black iron oxide (E172) and yellow iron oxide.

The most frequently encountered problem in oral dapoxetine formulations is the bitter taste. The tablets have typically been coated with coating agents, and mixtures of sweeteners or cation exchange resins have been used for masking the bitter taste.

On the other hand, phosphodiesterase type 5 inhibitors (PDE5 inhibitor) are used in the treatment of erectile dysfunction (ED). PDE5 inhibitors block the phosphodiesterase enzyme in a selective and efficient manner, thus increasing the level of cyclic guanosine monophosphate (cGMP) in the corpus cavernosum smooth muscle cells. Most frequently used PDE5 inhibitors are avanafil, lodenafil, mirodenafil, sildenafil, tadalafil, vardenafil, and udenafil. Tadalafil is a PDE5 inhibitor used in the treatment of ED and Pulmonary Arterial Hypertension (PAH). It has a longer half life as compared to other PDE5 inhibitors (mean, 17,5 hours). The chemical designation of tadalafil is (6R-trans)-6-(1,3-benzodioxol-5-yl)- 2,3,6,7,12,12a-hexahydro-2-methyl-pyrazino [1',2':1,6] pyrido[3,4-b]indole-1,4-dione, with the chemical structure illustrated below with Formula II.

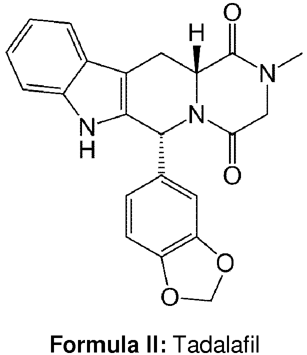

Formulations comprising a combination of selective serotonin reuptake inhibitors with PDE5 inhibitors are known in the prior art. The patent publication WO03000343, for example, discloses the use of a formulation comprising a combination of phosphodiesterase inhibitors and dapoxetine. On the other hand, MJ. Dresser et al. stated that dapoxetine, a selective serotonin reuptake inhibitor, does not have pharmaceutical interactions with PDE5 inhibitors and may be used for the treatment of premature ejaculation (Int J Impot Res. 2006 Jan-Feb;18(1):104-10).

The first object aimed with the tablet formulations comprising a combination of dapoxetine or a pharmaceutically acceptable salt thereof with tadalafil or a pharmaceutically acceptable salt thereof is to solve the problem stemming from the tadalafil active agent, i.e. to make the tablet highly bioavailable by providing a good disintegration and dissolution in the stomach. It is known that tadalafil is weakly soluble in water; this, in turn, causes a low dissolution rate in aqueous media such as the gastrointestinal liquid, and consequently, a low bioavailability is observed following oral digestion. Various methods have been used to overcome this problem. For instance, although that tadalafil is poorly soluble in water, it can be dissolved in organic solvents such as dimethylformamide and dimethylsulfoxide. In the patent publication EP1200091 B1, hydrophilic solvents such as polyethylene glycol 400, propylene glycol, and glycofurol are used to overcome the solubility problem of tadalafil. According to that patent, tadalafil is solved in hydrophilic solvents and then filled into soft capsules. According to the patent US6821975, tadalafil particles are micronized to overcome the solubility problem of tadalafil. The patent publication US20080009502, in turn, describes a solid composite comprising tadalafil and at least one hydrophilic carrier.

According to the prior art, particle size reduction is performed for water-insoluble drugs to increase the bioavailability thereof. Particle size reduction, however, is not always adequately efficient for increasing the dissolution rate to required levels. Strong aggregation tendencies are observed, yielding larger particles during the production process of many water-insoluble drugs; this, in turn, causes a general reduction in the effective surface area. Additionally, the flowability characteristics of drugs are influenced by the particle size, and extremely small sizes (lower than 10 µm) are not recommended for some active agents. (A. R. Gennaro, 'Remington's Pharmaceutical Sciences', 18th Edition, page 591, 1436-1437). The technical problem that may emerge as a result of particle size reduction is poor flowability and aggregation, since drug substances in powder form during production and tablet compression show less lubricant and glidant characteristics.

Therefore, an improved pharmaceutical formulation is needed comprising dapoxetine and tadalafil, which overcomes the problems of the related prior art and has high bioavailability and high solubility, is stable during the shelf life, is commercially less expensive, and is obtained by means of an environmentally-friendly production process. Other advantages and embodiments of the present invention will be clarified in the following description.

### Detailed Description of Invention

The main object of the present invention is to provide an improved oral tablet formulation of a combination of dapoxetine or a pharmaceutically acceptable salt thereof and tadalafil or a pharmaceutically acceptable salt thereof by making use of convenient excipients, which overcomes the problems mentioned above and is useful in the treatment of premature ejaculation and the associated symptoms thereof.

A further object of the present invention is to provide a simple, cost-effective, and timesaving process for preparing an improved oral tablet formulation of a combination of dapoxetine or a pharmaceutically acceptable salt thereof and tadalafil or a pharmaceutically acceptable salt thereof, which is easily dissolved in water and therefore has high absorption and high bioavailability.

In one embodiment, the tablet formulation comprises dapoxetine or a pharmaceutically acceptable salt thereof in an amount of 5.0 to 85.0% of the total weight of the formulation and tadalafil or a pharmaceutically acceptable salt thereof in an amount of 5.0 to 85.0% of the total weight of the formulation.

Tablet formulations according to the present invention should comprise appropriate proportions of suitable excipients for producing tablet formulations having high water-solubility and therefore high bioavailability. Surprisingly, it was observed that using dibasic calcium phosphate as a diluent together with crospovidone as a disintegrant in the formulation solves the problems mentioned above.

Dibasic calcium phosphate is not hygroscopic and therefore helps in obtaining a stable formulation. Dibasic calcium phosphate enhances the harmonization and flowability of the formulation, thereby providing convenience in the tablet compression step.

It was further observed that the flowability of a formulation comprising a combination of dapoxetine or a pharmaceutically acceptable salt thereof and tadalafil or a pharmaceutically acceptable salt thereof is brought to an outstanding level by making use of dibasic calcium phosphate. Dibasic calcium phosphate eliminates the difficulties encountered during the tablet compression step of a tablet formulation comprising tadalafil molecules of a small particle size. Furthermore, it was found that the use of crospovidone as a disintegrant together with dibasic calcium phosphate increases the efficiency of dibasic calcium phosphate.

In one embodiment, it was found that setting a suitable ratio dibasic calcium phosphate to crospovidone is effective in increasing the flowability and solubility of the tablet formulation. Accordingly, the ratio of the total weight of dibasic calcium phosphate to the total weight of crospovidone is in in the range of 100:1 to 1:1, preferably 70:1 to 10:1 and more preferably 50:1 to 5:1 to avoid the aforementioned problems.

In one embodiment of the present invention, the amount of dibasic calcium phosphate is 5.0 to 90.0% by weight, preferably 10.0 to 80.0% by weight, and more preferably 15.0 to 75.0% by weight.

Crospovidone features physical and chemical properties which make it an ideal disintegrant for the present invention. This is because crospovidone particles have a very distinct appearance from those of other disintegrants. Crospovidone particles look as if they are composed of agglomerates of mutually-fused smaller particles. This agglomeration imparts crospovidone a spongy and highly porous appearance, and even it takes water into its lattice very rapidly, it swells very slightly. This, in turn, helps crospovidone in dissolving easily and rapidly in a very low amount of water and provides crospovidone with a disintegration rate which is very higher than that of other relevant excipients.

As it is known in the prior art, reducing the particle size only of an active agent, which is poorly soluble in water, is not sufficient for increasing the solubility of that active agent. In our case, in turn, using crospovidone in a formulation comprising dapoxetine and tadalafil surprisingly increased the solubility of the formulation in the gastric juice.

According to one embodiment of the present invention, the amount of crospovidone is 1.0 to 40.0% by weight, preferably 3.0 to 35.0% by weight, and more preferably 5.0 to 30.0% by weight.

Another object of the present invention is to solve the problem which originates from the bitter taste of the dapoxetine active agent in a formulation comprising a combination of dapoxetine or a pharmaceutically acceptable salt thereof and tadalafil or a pharmaceutically acceptable salt thereof. Tablets comprising dapoxetine and tadalafil are coated with a humidity-barrier coating such that a tablet is obtained, which does not have a bitter taste and is stable throughout the shelf life.

In addition to the active agents, diluents, and disintegrants, a tablet formulation according to the present invention further comprises at least one pharmaceutically acceptable excipient which is selected from the group comprising lubricants, fillers, glidants, surfactants, and colorants.

It is known that magnesium stearate used in dapoxetine tablet formulations and in tadalafil tablet formulations has some drawbacks, and therefore it is used in small amounts in the production process of such drugs. Magnesium stearate is almost insoluble in water, and due to this hydrophobic character, the dissolution of a drug from a solid dosage form such as a tablet or capsule may be retarded. The dissolution of a tablet and particularly of a capsule is sensitive to both the amount of magnesium stearate present in the formulation and to the blending time thereof. The blending time should be limited. Long blending times may result in the formation of hydrophobic powder beds in the formulation which do not dissolve easily, whereas overblending can cause compaction problems. Tablet dissolution rate and crushing strength decrease as the time of blending increases; and magnesium stearate may also increase tablet friability. For this reason, blending times with magnesium stearate should be controlled carefully.

On the other hand, sodium stearyl fumarate is a highly efficient lubricant, being less hydrophobic than magnesium stearate and having a lower retarding effect on the dissolution of a tablet, as compared to magnesium stearate. Sodium stearyl fumarate does not either give rise to overblending-related problems, which is observed with magnesium stearate.

According to the embodiments above, a pharmaceutical composition of the present invention comprises sodium stearyl fumarate as a lubricant. In one embodiment of the present invention, the amount of sodium stearyl fumarate is 0.01 to 10.0% by weight, and preferably 0.1 to 5.0% by weight.

Suitable fillers include, but are not limited to, sugars, microcrystalline cellulose, mannitol, hydroxypropyl methylcellulose, hydroxypropyl cellulose, sodium carboxymethyl cellulose, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, or the mixtures thereof. According to the present invention, the amount of the filler(s) is 5.0 to 90.0% by weight, and preferably 10.0 to 80.0% by weight.

Suitable glidants include, but are not limited to, colloidal silicon dioxide, silica, calcium silicate, magnesium trisilicate, sodium stearyl fumarate, talk, and the mixtures thereof. According to the present invention, the amount of the glidant is 0.001 to 5.0% by weight. Suitable surfactants include, but are not limited to, sulfosuccinate, polysorbates and polyoxyethylene alkyl esters and ethers, glyceryl monolaurate saponins, sorbitan laurate, polyethylene lauryl ether, sodium lauryl sulfate, magnesium lauryl sulfate or mixture thereof. According to the present invention, the amount of the surfactants is 0.01 to 5.0% by weight, and preferably 0.1 to 2.0% by weight.

Suitable colorants include, but are not limited to, food, drug, and cosmetic (FD&C) dyes (e.g. FD&C blue, FD&C green, FD&C red, FD&C yellow, FD&C lake), ponceau, indigo drug & cosmetic (D&C) blue, indigotine FD&C blue, carmoisine indigotine (indigo carmine); iron oxides (e.g. iron oxide red, yellow, black), quinoline yellow, flame red, brilliant red (carmine), carmoisine, sunset yellow, etc., and the mixtures thereof.

A formulation according to the present invention can be produced by means of a direct compression method, a wet granulation method, or a dry granulation method. The preferred method is the direct compression method.

The present invention is described in more details in the following examples. These examples are not limiting the scope of the present invention and should be considered under the light of the foregoing detailed disclosure.

### Examples

**Example 1: A tablet formulation comprising dapoxetine and tadalafil**

| **Ingredients** | **Amount (%)** |
|---|---|
| Tadalafil | 5.00-85.00 |
| Dapoxetine | 5.00-85.00 |
| Crospovidone | 1.00-40.00 |
| Dibasic calcium phosphate | 5.00-90.00 |
| Sodium stearyl fumarate | 0.10-10.00 |
| Colloidal silicon dioxide | 0.10-0.20 |
| Microcrystalline cellulose | 5.00-90.00 |
| Hydroxypropyl methylcellulose | 5.00-90.00 |
| Polyethylene lauryl ether | 0.20-2.00 |
| Colorant | 0.10-6.00 |

### Production method: Direct compression method

Dapoxetine, tadalafil, crospovidone, dibasic calcium phosphate, microcrystalline cellulose, hydroxypropyl methyl cellulose, polyethylene lauryl ether and the colorant are mixed together. Then, the resulting mixture is mixed with colloidal silicon dioxide until a homogeneous mixture is obtained. After the resulting mixture is mixed with sodium stearyl fumarate for a short time, the tablet compression process is performed. A coating process is then performed using a humidity-barrier coating material, such as Opadry AMB/Kollicoat IR.

**Example 2: A tablet formulation comprising dapoxetine and tadalafil**

| **Ingredients** | **Amount (%)** |
|---|---|
| Tadalafil | 5.00-85.00 |
| Dapoxetine | 5.00-85.00 |
| Crospovidone | 1.00-40.00 |
| Dibasic calcium phosphate | 5.00-90.00 |
| Sodium stearyl fumarate | 0.10-10.00 |
| Silicon dioxide | 0.10-0.20 |
| Sodium carboxymethyl cellulose | 5.00-90.00 |
| Hydroxypropyl cellulose | 5.00-90.00 |
| Polyethylene lauryl ether | 0.20-2.00 |
| Colorant | 0.10-6.00 |

### Production method: Direct compression method

Dapoxetine, tadalafil, crospovidone, dibasic calcium phosphate, sodium carboxymethyl cellulose, hydroxypropyl cellulose, polyethylene lauryl ether and the colorant are mixed together. Then, the resulting mixture is mixed with silicon dioxide until a homogeneous mixture is obtained. After the resulting mixture is mixed with sodium stearyl fumarate for a short time, the tablet compression process is performed. A coating process is then performed using a humidity-barrier coating material, such as Opadry AMB/Kollicoat IR.

## Claims

1. An oral tablet formulation of dapoxetine or a pharmaceutically acceptable salt thereof and tadalafil or a pharmaceutically acceptable salt thereof, comprising crospovidone and dibasic calcium phosphate.

2. An oral tablet formulation according to Claim 1, wherein the weight ratio of crospovidone to dibasic calcium phosphate is in the range of 100:1 to 1:1, preferably 70:1 to 10:1 and more preferably 50:1 to 5:1.

3. An oral tablet formulation according to Claim 1, wherein the amount of crospovidone is from 1.0 to 40.0% by weight of the total tablet formulation, preferably from 3.0 to 35.0% by weight of the total tablet formulation, and more preferably from 5.0 to 30.0% by weight of the total tablet formulation.

4. An oral tablet formulation according to Claim 1, wherein the amount of dibasic calcium phosphate is from 5.0 to 90.0% by weight of the total tablet formulation, preferably from 10.0 to 80.0% by weight of the total tablet formulation, and more preferably from 15.0 to 75.0% by weight of the total tablet formulation.

5. An oral tablet formulation according to Claim 1, wherein the amount of dapoxetine or a pharmaceutically acceptable salt thereof is from 5.0 to 85.0% by weight of the total tablet formulation and the amount of tadalafil or a pharmaceutically acceptable salt thereof is from 5.0 to 85.0% by weight of the total tablet formulation.

6. An oral tablet formulation according to Claim 1, comprising a humidity-barrier film coating.

7. An oral tablet formulation according to any of the preceding claims 1 to 6, further comprising at least one pharmaceutically acceptable excipient which is selected from the group consisting of fillers, glidants, lubricants, surfactants, and colorants.

8. An oral tablet formulation according to Claim 7, wherein the lubricant is sodium stearyl fumarate.

9. An oral tablet formulation according to claims 7 and 8, wherein the amount of sodium stearyl fumarate is 0.01 to 10.0% by weight, and preferably 0.1 to 5.0% by weight.

10. An oral tablet formulation according to Claim 7, wherein the filler is selected from the group consisting of sugars, microcrystalline cellulose, hydroxypropyl cellulose, sodium carboxymethyl cellulose, mannitol, hydroxypropyl methylcellulose, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, or the mixtures thereof.

11. An oral tablet formulation according to Claim 10, wherein the amount of the filler is from 5.0 to 90.0% by weight of the total tablet formulation, and preferably from 10.0 to 80.0% by weight of the total tablet formulation.

12. An oral tablet formulation according to Claim 7, wherein the glidant is selected from the group consisting of colloidal silicon dioxide, silica, calcium silicate, magnesium trisilicate, sodium stearyl fumarate, talk or the mixtures thereof.

13. An oral tablet formulation according to Claim 12, wherein the amount of the glidant is from 0.001 to 5.0% by weight of the total tablet formulation.

14. An oral tablet formulation according to Claim 7, wherein the surfactant is selected from the group of consisting of dioctyl sulfosuccinate, polysorbates, polyoxyethylene alkyl esters and ethers, glyceryl monolaurate saponins, sorbitan laurate, polyethylene lauryl ether, sodium lauryl sulfate, magnesium lauryl sulfate, or the mixtures thereof.

15. An oral tablet formulation according to Claim 14, wherein the amount of the surfactant is from 0.01 to 5.0% by weight of the total tablet formulation, and preferably from 0.1 to 2.0% by weight of the total tablet formulation.

16. A method for preparing the oral tablet formulation of dapoxetine or a pharmaceutically acceptable salt thereof and tadalafil or a pharmaceutically acceptable salt thereof according to any of the preceding claims, comprising a direct compression method, a wet granulation method, or a dry granulation method and the method is preferably the direct compression method.

## Patentansprüche

1. Orale Tablettenformulierung von Dapoxetin oder einem pharmazeutisch verträglichen Salz davon und Tadalafil oder einem pharmazeutisch verträglichen Salz davon, umfassend Crospovidon und Calciumhydrogenphosphat.

2. Orale Tablettenformulierung nach Anspruch 1, wobei das Gewichtsverhältnis von Crospovidon zu Calciumhydrogenphosphat im Bereich von 100:1 bis 1:1, vorzugsweise 70:1 bis 10:1 und stärker bevorzugt 50:1 bis 5:1 liegt.

3. Orale Tablettenformulierung nach Anspruch 1, wobei die Menge an Crospovidon von 1,0 bis 40,0 Gew.-% der gesamten Tablettenformulierung, vorzugsweise von 3,0 bis 35,0 Gew.-% der gesamten Tablettenformulierung und stärker bevorzugt von 5,0 bis 30,0 Gew.-% der gesamten Tablettenformulierung beträgt.

4. Orale Tablettenformulierung nach Anspruch 1, wobei die Menge an Calciumhydrogenphosphat von 5,0 bis 90,0 Gew.-% der gesamten Tablettenformulierung, vorzugsweise von 10,0 bis 80,0 Gew.-% der gesamten Tablettenformulierung und stärker bevorzugt von 15,0 bis 75,0 Gew.-% der gesamten Tablettenformulierung beträgt.

5. Orale Tablettenformulierung nach Anspruch 1, wobei die Menge an Dapoxetin oder die Menge eines pharmazeutisch verträglichen Salzes davon von 5,0 bis 85,0 Gew.-% der gesamten Tablettenformulierung beträgt und die Menge an Tadalafil oder die Menge eines pharmazeutisch verträglichen Salzes davon von 5,0 bis 85,0 Gew.-% der gesamten Tablettenformulierung beträgt.

6. Orale Tablettenformulierung nach Anspruch 1, umfassend einen Feuchtigkeitsbarriere-Filmüberzug.

7. Orale Tablettenformulierung nach einem der vorangehenden Ansprüche 1 bis 6, weiterhin umfassend mindestens einen pharmazeutisch verträglichen Exzipienten, der ausgewählt ist aus der Gruppe bestehend aus Füllstoffen, Fließregulierungsmitteln, Gleitmitteln, grenzflächenaktiven Stoffen und Farbstoffen.

8. Orale Tablettenformulierung nach Anspruch 7, wobei das Gleitmittel Natriumstearylfumarat ist.

9. Orale Tablettenformulierung nach den Ansprüchen 7 und 8, wobei die Menge an Natriumstearylfumarat 0,01 bis 10,0 Gew.-% und vorzugsweise 0,1 bis 5,0 Gew.-% beträgt.

10. Orale Tablettenformulierung nach Anspruch 7, wobei der Füllstoff ausgewählt ist aus der Gruppe bestehend aus Zuckern, mikrokristalliner Cellulose, Hydroxypropylcellulose, Natriumcarboxymethylcellulose, Mannitol, Hydroxypropylmethylcellulose, Sorbitol, Saccharose, anorganischen Salzen, Calciumsalzen, Polysacchariden, Glucose, Calciumhydrogenphosphat, Natriumchlorid, Dextraten, Lactitol, Maltodextrin, Saccharose-Maltodextrin-Gemischen, Xylitol, Trehalose, schwerem Magnesiumcarbonat, oder den Gemischen davon.

11. Orale Tablettenformulierung nach Anspruch 10, wobei die Menge des Füllstoffs von 5,0 bis 90,0 Gew.-% der gesamten Tablettenformulierung und vorzugsweise von 10,0 bis 80,0 Gew.-% der gesamten Tablettenformulierung beträgt.

12. Orale Tablettenformulierung nach Anspruch 7, wobei das Fließregulierungsmittel ausgewählt ist aus der Gruppe bestehend aus kolloidalem Siliciumdioxid, Siliciumdioxid, Calciumsilicat, Magnesiumtrisilicat, Natriumstearylfumarat, Talkum oder den Gemischen davon.

13. Orale Tablettenformulierung nach Anspruch 12, wobei die Menge des Fließregulierungsmittels von 0,001 bis 5,0 Gew.-% der gesamten Tablettenformulierung beträgt.

14. Orale Tablettenformulierung nach Anspruch 7, wobei der grenzflächenaktive Stoff ausgewählt ist aus der Gruppe bestehend aus Dioctylsulfosuccinat, Polysorbaten, Polyoxyethylenalkylestern und -ethern, Glycerylmonolaurat, Saponinen, Sorbitanlaurat, Polyethylenlaurylether, Natriumlaurylsulfat, Magnesiumlaurylsulfat, oder den Gemischen davon.

15. Orale Tablettenformulierung nach Anspruch 14, wobei die Menge des grenzflächenaktiven Stoffes von 0,01 bis 5,0 Gew.-% der gesamten Tablettenformulierung und vorzugsweise von 0,1 bis 2,0 Gew.-% der gesamten Tablettenformulierung beträgt.

16. Verfahren zur Herstellung der oralen Tablettenformulierung von Dapoxetin oder einem pharmazeutisch verträglichen Salz davon und Tadalafil oder einem pharmazeutisch verträglichen Salz davon nach einem der vorangehenden Ansprüche, umfassend ein Direktverpressungsverfahren, ein Feuchtgranulierungsverfahren oder ein Trockengranulierungsverfahren, und das Verfahren ist vorzugsweise das Direktverpressungsverfahren.

## Revendications

1. Formulation de comprimé oral de dapoxétine ou d'un sel pharmaceutiquement acceptable de celle-ci et de tadalafil ou d'un sel pharmaceutiquement acceptable de celui-ci, comprenant de la crospovidone et du phosphate de calcium dibasique.

2. Formulation de comprimé oral selon la revendication 1, dans laquelle le rapport en poids de la crospovidone au phosphate de calcium dibasique se situe dans la plage de 100:1 à 1:1, de préférence de 70:1 à 10:1, et de façon davantage préférée de 50:1 à 5:1.

3. Formulation de comprimé oral selon la revendication 1, dans laquelle la quantité de crospovidone est de 1,0 à 40,0 % en poids de la formulation de comprimé totale, de préférence de 3,0 à 35,0 % en poids de la formulation de comprimé totale, et de façon davantage préférée de 5,0 à 30,0 % en poids de la formulation de comprimé totale.

4. Formulation de comprimé oral selon la revendication 1, dans laquelle la quantité de phosphate de calcium dibasique est de 5,0 à 90,0 % en poids de la formulation de comprimé totale, de préférence de 10,0 à 80,0 % en poids de la formulation de comprimé totale, et de façon davantage préférée de 15,0 à 75,0 % en poids de la formulation de comprimé totale.

5. Formulation de comprimé oral selon la revendication 1, dans laquelle la quantité de dapoxétine ou d'un sel pharmaceutiquement acceptable de celle-ci est de 5,0 à 85,0 % en poids de la formulation de comprimé totale et la quantité de tadalafil ou d'un sel pharmaceutiquement acceptable de celui-ci est de 5,0 à 85,0 % en poids de la formulation de comprimé totale.

6. Formulation de comprimé oral selon la revendication 1, comprenant un enrobage de film barrière à l'humidité.

7. Formulation de comprimé oral selon l'une quelconque des revendications précédentes 1 à 6, comprenant en outre au moins un excipient pharmaceutiquement acceptable qui est choisi dans le groupe consistant en les charges, les agents glissants, les lubrifiants, les tensio-actifs et les colorants.

8. Formulation de comprimé oral selon la revendication 7, dans laquelle le lubrifiant est le fumarate de sodium et de stéaryle.

9. Formulation de comprimé oral selon l'une des revendications 7 et 8, dans laquelle la quantité de fumarate de sodium et de stéaryle est de 0,01 à 10,0 % en poids, et de préférence de 0,1 à 5,0 % en poids.

10. Formulation de comprimé oral selon la revendication 7, dans laquelle la charge est choisie dans le groupe consistant en les sucres, la cellulose microcristalline, l'hydroxypropyl cellulose, la carboxyméthyl cellulose sodique, le mannitol, l'hydroxypropyl méthylcellulose, le sorbitol, le sucrose, les sels inorganiques, les sels de calcium, les polysaccharides, le dextrose, le phosphate dicalcique, le chlorure de sodium, les dextrates, le lactitol, la maltodextrine, les mélanges sucrose-maltodextrine, le xylitol, le tréhalose, le carbonate de magnésium lourd ou leurs mélanges.

11. Formulation de comprimé oral selon la revendication 10, dans laquelle la quantité de la charge est de 5,0 à 90,0 % en poids de la formulation de comprimé totale et, de préférence de 10,0 à 80,0 % en poids de la formulation de comprimé totale.

12. Formulation de comprimé oral selon la revendication 7, dans laquelle l'agent glissant est choisi dans le groupe consistant en le dioxyde de silicium colloïdal, la silice, le silicate de calcium, le trisilicate de magnésium, le fumarate de sodium et de stéaryle, le talc ou les mélanges de ceux-ci.

13. Formulation de comprimé oral selon la revendication 12, dans laquelle la quantité de l'agent glissant est de 0,001 à 5,0 % en poids de la formulation de comprimé totale.

14. Formulation de comprimé oral selon la revendication 7, dans laquelle le tensio-actif est choisi dans le groupe consistant en le sulfosuccinate de dioctyle, les polysorbates, les alkyl esters et éthers polyoxyéthylénés, le monolaurate de glycéryle, les saponines, le laurate de sorbitan, le lauryl éther polyoxyéthyléné, le lauryl sulfate de sodium, le lauryl sulfate de magnésium ou les mélanges de ceux-ci.

15. Formulation de comprimé oral selon la revendication 14, dans laquelle la quantité du tensio-actif est de 0,01 à 5,0 % en poids de la formulation de comprimé totale, et, de préférence, de 0,1 à 2,0 % en poids de la formulation de comprimé totale.

16. Procédé de préparation de la formulation de comprimé oral de dapoxétine ou d'un sel pharmaceutiquement acceptable de celle-ci et de tadalafil ou d'un sel pharmaceutiquement acceptable de celui-ci selon l'une quelconque des revendications précédentes, comprenant un procédé de compression directe, un procédé de granulation par voie humide ou un procédé de granulation par voie sèche et le procédé est de préférence le procédé de compression directe.
